# FASCICULE DE BREVET EUROPEEN

(11) **EP 2 180 906 B1**
(45) Date de publication et mention de la délivrance du brevet: **24.12.2014**
(21) Numéro de dépôt: 08803456.6
(22) Date de dépôt: 01.09.2008
(51) Int. Cl.: A61L 27/20, A61L 27/36, A61L 27/56, A61L 27/38

(54) **PROTHESE DESTINEE A PROMOUVOIR LA RECONSTRUCTION IN VIVO D'UN ORGANE CREUX OU D'UNE PARTIE D'ORGANE CREUX**
PROTHESE ZUR FÖRDERUNG DER IN-VIVO-REKONSTRUKTION EINES HOHLORGANS ODER EINES TEILS EINES HOHLORGANS
PROSTHESIS FOR PROMOTING THE IN VIVO RECONSTRUCTION OF A HOLLOW ORGAN OR A PORTION OF A HOLLOW ORGAN

(30) Priorité: 31.08.2007 FR 0757308
(43) Date de publication de la demande: 05.05.2010
(73) Titulaire: Synolyne Pharma, 4040 Herstal (BE)
(72) Inventeur: MAQUET, Véronique, B-4257 Berloz (BE); GAUTIER, Sandrine, B-4000 Liege (BE); COULIC, Véry, B-6001 Marcinelle (BE)
(74) Mandataire: Jacobson, Claude
(86) Numéro de dépôt international: PCT/EP2008/061473
(87) Numéro de publication internationale: WO 2009/027537

(56) Documents cités:
- EP-A- 0 230 635
- EP-A- 0 393 788
- WO-A-2004/072280
- WO-A-2006/047758
- US-A1- 2004 193 242
- US-A1- 2004 198 584

## Description

La présente invention concerne une nouvelle prothèse destinée à promouvoir la reconstruction in vivo d'un organe creux ou d'une partie d'un tel organe.

Elle concerne plus précisément une bioprothèse pour la reconstruction in vivo d'un organe creux, humain ou animal ou d'une partie d'un tel organe.

### ETAT DE L'ART

Le remplacement de tissus creux et en particulier celui de défauts circulaires de tels organes et plus précisément de l'oesophage reste un des problèmes les plus difficiles en chirurgie, notamment en chirurgie digestive.

Jusqu'aux années 50, des segments d'intestins et d'estomacs autologues (prélevés sur le patient lui-même) ont été largement utilisés pour remplacer des organes creux comme des segments d'oesophage, de cholédoque, la vessie, l'urètre ou pour la recanalisation des trompes de Fallope. Ces autogreffes sont toutefois associées à un pourcentage élevé de complications postopératoires.

Dans les années 60, grâce au développement des polymères, des prothèses polymères ont été largement utilisées pour différentes applications (oesophage, estomac, conduit biliaire, vaisseaux sanguins). Ces prothèses sont faites à partir de différents matériaux (polyéthylène, silicone, polyuréthane, terpolymère acrylate-amide, polytétrafluoroéthylène). Les prothèses polymères sont habituellement bien tolérées mais leur intégration n'est pas optimale. La prothèse n'étant en contact avec les tissus vivants que sur une seule face, sa colonisation ne se fait pas, ce qui résulte en la formation d'escarre, à son détachement et son élimination. Quelle que soit leur composition, ces prothèses sont donc seulement temporaires et doivent être remplacées régulièrement. Leur utilisation clinique est donc limitée à certaines applications comme le drainage du cholédoque, les conduits pancréatiques, les tubes de la trachée et de l'oesophage.

L'espérance de vie des patients atteints de cancer avancé de l'oesophage est très courte et la prise en charge de la plupart de ces patients est limitée à des traitements palliatifs : résection chirurgicale, radio/chimiothérapie, avec des résultats très médiocres. Bien que le recours à des prothèses se soit démontré efficace pour résoudre la dysphasie et améliorer la qualité de vie des patients atteints du cancer de l'oesophage, des complications comme la migration, la perforation et l'obstruction par la nourriture mènent à des mortalités trop élevées.

Des bioprothèses ont été conçues pour recouvrir la surface interne de tubes artificiels avec des cultures de tissus. Cette option s'est avérée efficace pour le remplacement de lésions partielles, sous forme de sorte de pansements, souvent appelés "patchs" mais n'ont pas été utilisés pour résoudre des lésions circulaires de l'oesophage.

L'utilisation de stents métalliques expansibles est considérée comme une alternative efficace aux tubes plastiques non expansibles mais reste grevée des mêmes complications (escarres, élimination).

Les patchs représentent une solution thérapeutique efficace pour le traitement de lésions partielles qui n'entament pas toute la circonférence de l'organe (par exemple après ablation de verticules). Cependant à l'heure actuelle, les options thérapeutiques pour guérir les lésions circulaires comme celles apparaissant au niveau de l'oesophage suite à un cancer ou à une brûlure avec sténose grave sont très limitées et devraient reposer sur une meilleure conception de la prothèse oesophagienne.

Des stratégies basées sur des polymères biodégradables sont apparues comme une alternative dans le but de développer ou régénérer des nouveaux tissus. De nombreux matériaux biodégradables sous forme d'éponges, de tissus maillés (appelés « meshs », ou matrices dans certains cas), de tubes et de nanofibres ont été utilisés, chez le rat ou la souris, comme support de régénération d'oesophage, sur des modèles expérimentaux. Pour ce faire ont été utilisés des polymères synthétiques comme les polyesters biodégradables de la famille de l'acide polylactique (PLA), polyglycolique (PGA) et de la polycaprolactone (PCL). Certains de ces produits sont disponibles commercialement (ex : mailles chirurgicales Vicryl (« Vicryl surgical mesh»). Cependant, ces polymères synthétiques seuls ne sont pas capables d'induire la réponse biologique menant à la régénération de tissus, par manque de bio-mimétisme, ce qui nécessite souvent le recours à des modifications de surface (greffage de collagène, fibronectine).

La transplantation de tissus adultes (muqueuse et sous-muqueuse jujénales, greffes iléales revascularisée, dure-mère lyophilisée) et de matériels autologues (cellules, muqueuses) a été décrite pour traiter des lésions de l'oesophage.

Cependant, les tissus adultes ne supportent pas l'ischémie ce qui limite leur chance de survie après transplantation. Les greffes de tissus adultes sont également pénibles, invalidantes et nécessitent des interventions répétées. Elles constituent une option pour la fermeture des défauts non circulaires mais pas pour les lésions circulaires.

La transposition totale de l'estomac a également été décrite mais elle génère des problèmes comme le reflux, et l'évacuation trop rapide vers l'intestin.

Des stratégies combinées basées sur l'utilisation de prothèses synthétiques combinées à des cellules autologues plutôt qu'à des tissus adultes ont été proposées.

Ce concept appelé ingénierie tissulaire a soulevé un grand intérêt, ces 10 dernières années.

Il repose sur l'utilisation d'un support polymère biodégradable naturel ou synthétique combiné à des cellules humaines de préférence autologues, préalablement cultivées in vitro. L'ensemble cellules-matrices et ensuite implanté in vivo dans le but de reconstruire, de régénérer ou de réparer un organe ou un tissu lésé. Cette stratégie a été développée par Marzaro et al. (Journal of Biomedical Materials Research, 2006, 77a(4), 795-801) qui a proposé l'utilisation d'une matrice acellulaire d'oesophage homologue et des cellules musculaires lisses autologues in vitro pour le développement d'un oesophage implantable.

Des tubes bicouches composés de réseaux de collagène ensemencés et d'une couche de muscle ont également été fabriqués pour l'ingénierie de l'oesophage. Ils ont permis l'infiltration cellulaire et la néo-vascularisation.

On a aussi utilisé un oesophage décellularisé comme support biocompatible pour l'ingénierie tissulaire. Cependant, cette option soulève le problème de la disponibilité des tissus ainsi que la mise en oeuvre de la prothèse pour correspondre à la taille, à la dimension de l'organe à réparer. De plus, les supports artificiels et les tissus autologues utilisés pour la reconstruction de l'oesophage peuvent induire des complications telles que la sténose et la fuite à long terme car leur surface interne ne peut entièrement se couvrir d'épithélium.

La demande de brevet chinois CN 1410034 A décrit un support présentant une structure en bicouche composée d'une peau et d'une couche sous-dermique contenant des cellules vasculaires pour l'ingénierie tissulaire de l'oesophage. Le support peut être un biomatériau ou un matériel synthétique mais est associé de préférence avec une matrice acellulaire. Les cellules ensemencées peuvent être des fibrocytes, des cellules endothéliales ou des kératinocytes. Le dispositif pourrait être utilisé pour la régénération de tissu mais pas pour le remplacement d'un organe complet tel que l'oesophage.

Tous les autres cas de thérapie existant actuellement présentent des problèmes de récolte de cellules, de temps de conception et un coût élevé.

La demande de brevet WO 2006/047758 décrit une manière de préparer des tubes de chitosane comprenant une couche poreuse et une couche non poreuse. Cette demande de brevet décrit l'utilisation de la force centrifuge comme moyen de réalisation d'une structure tubulaire, et positionne inévitablement la couche non poreuse sur la surface externe de la structure tubulaire. Cette demande ne décrit donc pas une structure tubulaire permettant de résoudre le problème technique de reconstruire un organe tubulaire. D'autre part, aucun exemple de reconstruction tissulaire n'est donné.

### BUT DE L'INVENTION

L'invention a pour but de résoudre l'ensemble des problèmes décrits ci-dessus, notamment par la fourniture d'une nouvelle prothèse destinée à promouvoir la reconstruction d'un organe creux ou une partie d'un organe creux.

Dans ce cadre l'invention se propose de résoudre le problème technique de supporter les mouvements imposés à l'organe à reconstruire, et notamment à l'oesophage qui se situe en partie dans une zone du corps régulièrement mis en mouvement (torsion, déglutition, etc.) : le cou.

D'autre part, l'invention a pour but de résoudre le problème consistant en fournir une prothèse ayant des propriétés adéquates pour reconstruire l'organe, comme la résistance mécanique et/ou l'étanchéité de la prothèse à un fluide corporel en contact permanent ou non avec la surface interne de l'organe à reconstruire.

### DESCRIPTION DE L'INVENTION

Le chitosane est un biopolymère obtenu par déacétylation de la chitine, présente dans la paroi des crustacés, la cuticule des arthropodes, les endosquelettes des céphalopodes, les diatomées, ou encore d'origine fongique comme dans la parois des champignons. Il possède des propriétés intéressantes incluant la biocompatibilité, la biodégradabilité, une structure analogue aux glycosaminoglycanes de la matrice extra-cellulaire. Le chitosane est de grand intérêt pour les applications biomédicales incluant la cicatrisation, les systèmes de libération contrôlée de médicaments, les dispositifs hémostatiques, les applications chirurgicales (fils de sutures résorbables, barrières anti adhésives), l'ophtalmologie, les applications d'ingénierie tissulaire, d'encapsulation cellulaire, de thérapie génique et de vaccination.

Une revue des applications potentielles du chitosane a été publiée par Khor et ses collaborateurs [Khor and Lim, Biomater 2003;24:2339-2349].

Le chitosane convient pour l'ingénierie tissulaire des organes creux conçus lorsqu'il se trouve sous forme de structure cellulaire poreuse. Dans "Chitin-based tubes for tissue engineering in the nervous system », Biomater 2005;26-4624-4632, Freier et ses collaborateurs ont rapporté une méthode de préparation de tubes de chitosane obtenus à partir d'une hydrolyse alcaline de la chitine. Les auteurs ont montré une cyto-compatibilité des films de chitosane avec les neurones issus de ganglions des racines dorsales et la croissance neurale in vitro.

WO 2007/042281 A2, décrit une méthode basée sur un procédé d'extraction d'un gel de N-acylchitosane pour la construction de fibres et de tubes de chitosane et de dérivés de chitosane ayant une résistance mécanique suffisante, sans utilisation de solvants toxiques ou d'agents de réticulation et autres composés toxiques.

Les méthodes de préparation de structures spécifiques contenant du chitosane telles que les tubes creux à structure poreuse ont été décrites par Madihally et ses collaborateurs [Madihally and Matthew, Biomater 1999; 20:1133-1142]. Les supports en tube poreux sont préparés par congélation d'une solution de chitosane contenue dans des tubes plastiques cylindriques. Des tubes avec une membrane luminale non poreuse peuvent être obtenus par un premier enrobage du tube inerte avec un film de chitosane le dit film étant obtenu par la gélification du chitosane en milieu basique suivi de sa déshydratation à l'air. Après séchage et réhydratation du support à l'aide d'un traitement à la soude ou à l'éthanol ainsi qu'une neutralisation avec un tampon salin phosphate, le support est caractérisé par microscopie électronique et par des tests mécaniques. Plusieurs évaluations biologiques du support ont été effectuées, mais ce document ne présente aucun résultat technique dans une application en ingénierie tissulaire.

La faisabilité d'utilisation des matériaux à base de chitosane pour développer un oesophage en ingénierie tissulaire a été étudiée par Qin et ses collaborateurs (Qin, Xiong, Duier Junyi Daxue Xuebao 2002, 23, 1134-1137) qui ont implanté des membranes de collagène-chitosane avec des cellules épithéliales d'oesophage de rats de manière sous-musculaire. Ils ont montré que l'ensemble greffé reste sain après 2 semaines et est complètement dégradé après 4 semaines suivant l'implantation. Les auteurs ont montré une comptabilité cellulaire du support polymérique mais ne donnent pas d'autres descriptions du support, et notamment ne décrivent pas l'utilisation de ce support en tant qu'oesophage artificiel biodégradable.

Au vu des problèmes posés par la reconstruction des organes creux et, tout particulièrement de l'oesophage, surtout lorsqu'ils sont atteints de défauts circulaires, il apparaît qu'il existe un réel besoin de développer de nouvelles solutions susceptibles de permettre la régénération de ces organes, surtout lorsqu'ils sont atteints de défauts circulaires.

La présente invention propose un nouveau type de prothèse ou bioprothèse pouvant être implantée dans ou sur, ou en relation avec un organe creux et, tout particulièrement l'oesophage, en vue d'assurer sa régénération.

Cette bioprothèse comprend un support poreux biodégradable associé avec un matériel vivant de préférence peu ou pas différencié et, de préférence un matériel foetal, ou associé avec des cellules du tissu de l'organe à reconstruire.

Le support poreux biodégradable est avantageusement constitué de chitosane mais peut être également constitué de tout matériau polymère biodégradable et biocompatible susceptible d'être mis en oeuvre pour conduire à la porosité recherchée. Une combinaison de différents supports poreux est également couverte par la présente invention (comme par exemple chitosane/collagène, chitosane/glycosaminoglycanes comme les combinaisons chitosane/acide hyaluronique, ou toute autre combinaison bien connue de l'homme du métier).

Le support tubulaire biodégradable est conçu de façon originale pour présenter :
- une surface externe poreuse biodégradable, permettant la prolifération cellulaire et la vascularisation des cellules,
- une surface interne non poreuse biodégradable, en contact avec le bol alimentaire dans le cas où il s'agit d'une prothèse d'oesophage ou, plus généralement, avec un fluide corporel circulant dans un organe creux,
- un diamètre et des proportions qui sont les mêmes que ou équivalents à ceux de l'organe à reconstituer,
- des propriétés mécaniques suffisantes.

Cette prothèse présente l'avantage de pouvoir être réalisée facilement à partir d'un biopolymère biocompatible et biodégradable et de respecter les propriétés anatomiques des différents organes. De manière surprenante cette prothèse permet une excellente reconstruction dirigée de l'organe creux, ou partie d'organes creux, à reconstruire ou à remplacer. On entend par reconstruction dirigée, une reconstruction de l'organe ou d'une partie de l'organe par une prolifération cellulaire au sein de la prothèse. Il est notamment surprenant que les cellules, implantées via le matériau biologique ajouté, soient capables de proliférer et soient fonctionnelles pour permettre la reconstruction de la matrice extracellulaire, bien qu'étant en présence du support tubulaire biodégradable, et ainsi reconstruire la partie remplacée de l'organe creux. D'autre part on obtient les propriétés mécaniques et physiologiques recherchées.

Le support tubulaire est utilisé de préférence en combinaison avec un matériel vivant différencié ou peu ou pas différencié. Selon une variante on utilise un matériel foetal spécifique de l'organe à réparer, permettant ainsi la régénération de l'organe en respectant la structure, la morphologie et la fonction de l'organe considéré. Selon une autre variante on utilise la matière vivante constituée par au moins une partie des cellules du tissu de l'organe à reconstruire. Ces cellules sont généralement les cellules ayant une bonne capacité de prolifération au sein de la couche poreuse de l'invention.

La présente invention concerne donc une bioprothèse complexe composée a) d'un support tubulaire poreux biocompatible et bio dégradable, et b) d'un matériel vivant peu ou pas différencié et, de préférence, un matériel foetal spécifique permettant la formation d'une matrice ectopique de matériel foetal après l'implantation.

Un des objectifs de la présente invention est de produire une bioprothèse complexe pour la régénération d'organes creux pour pallier les limitations de l'état de l'art lié à l'utilisation de cellules autologues ou de tissus adultes. Cette bioprothèse permet la régénération de l'organe entier sans risques de transmissions virales ou rejets de greffes. (En cas d'utilisation de matériel foetal allo- ou xénogénique (donneur de souches ou d'espèces différentes de celle du receveur), les mesures habituelles d'immunodépression ou de création de tolérance doivent être envisagées)

La présente invention concerne, selon un premier aspect, une prothèse destinée à promouvoir la reconstruction in vivo d'un organe creux ou d'une partie d'un organe creux, caractérisée en ce qu'elle comprend :
- un support tubulaire creux biodégradable comprenant au moins un matériau polymère biocompatible et biodégradable, ledit support comprenant une couche externe poreuse et une couche interne essentiellement non poreuse et,
- un matériau d'origine biologique vivante à la surface externe, et/ou au sein d'au moins une partie de la couche poreuse dudit support, et/ou sur la surface de la couche essentiellement non poreuse faisant face à la couche poreuse, ledit matériau d'origine biologique étant choisi pour permettre la reconstruction in vivo dudit organe ou de ladite partie d'organe.

L'invention couvre des variantes dans lesquelles la couche essentiellement non poreuse et la couche poreuse sont constituées de matériaux différents, mais également les variantes dans lesquelles ces couches sont constituées de matériaux identiques bien que leur porosité soit différente.

Selon un deuxième aspect, l'invention concerne également un procédé de fabrication d'une telle prothèse. Ce procédé comprend la préparation d'un support tubulaire poreux comprenant une couche essentiellement non poreuse sur sa face interne et l'incorporation à la surface externe dudit support tubulaire et/ou en son sein d'un matériau d'origine biologique. Le procédé comprend en particulier la préparation d'un support tubulaire biodégradable comprenant une couche externe poreuse permettant une prolifération cellulaire et une couche interne essentiellement non poreuse (ne permettant sensiblement pas de prolifération cellulaire), et l'incorporation d'un matériel biologique destiné à former prothèse à la surface externe, et/ou au sein d'au moins une partie de la couche poreuse dudit support, et/ou sur la surface de la couche essentiellement non poreuse faisant face à la couche poreuse.

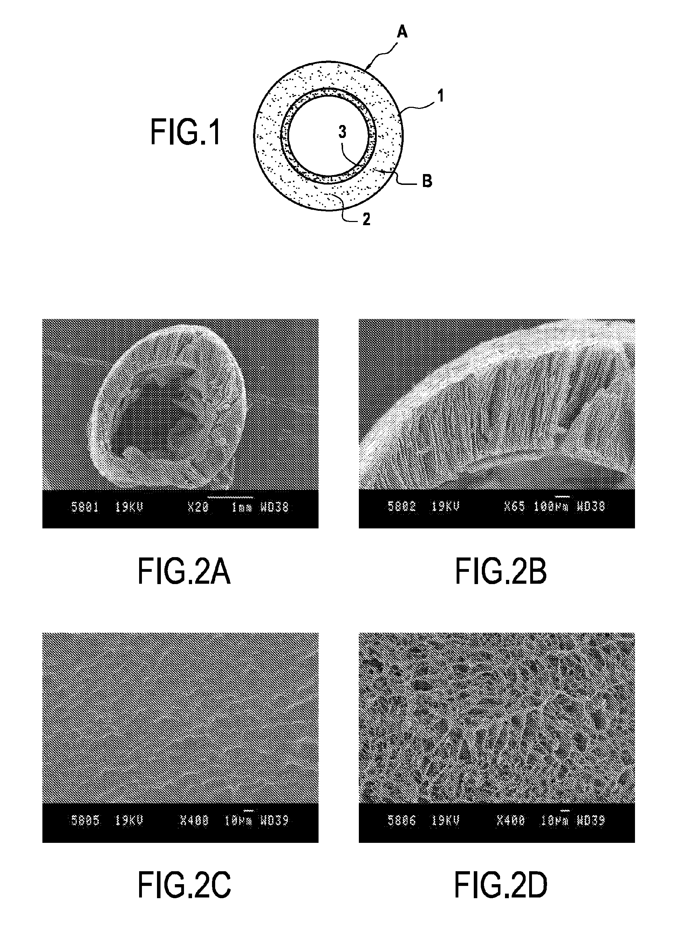

D'autres caractéristiques et avantages de la présente invention ressortent de la description détaillée et des exemples qui suivent illustrés par les figures 1 à 8.

La figure 1 est un schéma d'un tube de l'invention vu en coupe. Sur la figure 1, la référence 1 désigne la couche aux externes du tube comprenant le polymère biodégradable, la référence 2 désigne l'intervalle pour le développement des cellules et de l'organe vivant, et la référence 3 désigne la couche essentiellement non poreuse interne. La référence A localise le matériel vivant dans un premier mode de réalisation dans lequel le matériel vivant est placé ou fixé à la surface externe du tube. La référence B localise le matériel vivant dans un second mode de réalisation dans lequel le matériel vivant est placé entre la couche externe poreuse et la couche non poreuse.
La figure 2 constituée des figures 2A, 2B, 2C et 2D présente des clichés obtenus par microscopie électronique à balayage correspondant au tube poreux obtenu selon l'exemple 1.
Les figures 3A, 3B et 3C concernent les étapes de l'intégration puis de la résorption du matériel chitosane dans l'organisme à 7 (a,b) et 14 (c) jours après son implantation.
Les figures 4A et 4B montrent le développement ectopique d'un intestin foetal à 2 et 3 mois en présence d'un tube de chitosane et la disparition (résorption) de celui-ci.
Les figures 5A, 5B, 5C et 5D représentent schématiquement une méthode pour insérer une variante du support tubulaire de l'invention, où la couche interne non poreuse et la couche externe poreuse sont physiquement indépendantes.
Les figures 6A, 6B, 6C, 7A, 7B, et 7C représentent schématiquement une méthode pour insérer une variante du support tubulaire de l'invention, où la couche interne non poreuse et la couche externe poreuse sont physiquement indépendantes.
Les figures 8A, 8B, et 8C représentent schématiquement des variantes de l'invention comprenant un moyen favorisant la flexibilité du support tubulaire.

L'invention concerne une bioprothèse combinée pour la régénération d'organes creux et plus particulièrement pour la régénération de parties d'oesophage présentant une pathologie. D'autres organes peuvent être réparés, remplacés ou régénérés en utilisant la présente invention tels que l'intestin, le cholédoque, l'estomac, le conduit pancréatique, les conduits urinaires (urètre et uretère) et la vessie, les vaisseaux sanguins, les trompes de Fallope et l'utérus. La pathologie peut être par exemple un cancer. Ainsi l'invention couvre l'utilisation de la prothèse selon la présente invention, y compris toutes ses variantes, pour remplacer un organe creux dont une partie au moins est atteinte d'un cancer, et en particulier lorsque l'organe creux est l'oesophage. L'invention couvre donc également les méthodes de traitement chirurgical d'un cancer d'au moins une partie d'un organe creux, notamment dans le cas où la découpe ou l'ablation d'une section entière de l'organe creux est nécessaire. D'autres pathologies pourront également bénéficier de ce traitement comme par exemple les brûlures avec sténose grave. Ainsi l'invention concerne une méthode de traitement chirurgical d'une pathologie nécessitant découpe ou l'ablation d'au moins une partie d'une section d'un organe tubulaire creux, caractérisée en ce qu'elle comprend découpe ou l'ablation d'une section totale ou partielle d'un organe creux, et le positionnement à proximité de la zone ayant subi découpe ou l'ablation d'une prothèse, d'un support tubulaire, ou d'un matériau polymère défini dans l'invention, y compris toutes les variantes, pour reconstruire in vivo la partie découpée ou ablatée.

En accord avec la présente invention, le support tubulaire est avantageusement construit en un polymère biocompatible et biodégradable. Le tube est poreux mais associé à une surface inerte essentiellement non poreuse au niveau de sa paroi interne. Il est avantageux que les couches poreuse et essentiellement non poreuse soient constituées du même polymère biodégradable.

En accord avec la présente invention, le support tissulaire montre des propriétés mécaniques suffisantes, compatibles avec les conditions mécaniques rencontrées in vivo.

Selon une variante, le support tubulaire de l'invention comprend un moyen favorisant la flexibilité du support, notamment pour améliorer la résistance au mouvement de l'organe reconstruite. Ces moyens sont par exemple une structure en accordéon, ou en spirale, sans y être limités.

En accord avec la présente invention, le support tubulaire est compatible avec le matériel biologique, et de préférence du matériel foetal.

En accord avec la présente invention, le support tubulaire est biodégradable in vivo et a une dégradation contrôlée dans le temps donnant un support temporaire permettant la croissance et la prolifération de néo-tissus.

En accord avec la présente invention, le support tubulaire présente une structure et des dimensions spécifiques en accord avec l'anatomie et la fonction des organes à reconstituer. La couche essentiellement non-poreuse doit assurer l'étanchéité de la couche poreuse et/ou de la matière vivante vis-à-vis du milieu biologique pouvant être contenu ou passant dans l'organe à reconstruire (liquide biologique, bol alimentaire, etc). Il est entendu par étanchéité, l'absence de passage de substances pouvant détériorer le fonctionnement de l'hôte, voire une inflammation ne pouvant pas naturellement se résorber dans le temps. La couche essentiellement non poreuse peut avoir une épaisseur comprise entre 60 µm et 3 mm, ou au maximum de 2,5 ou 2 mm. Elle peut aussi être d'au moins 100 µm d'épaisseur. La couche essentiellement non poreuse sert également de support soit pour la matière vivante selon une variante de l'invention, soit pour la couche poreuse contenant éventuellement la matière vivante selon une autre variante. La couche non poreuse peut servir également de guide pour la reconstruction de l'organe tubulaire creux, comme l'oesophage.

En accord avec la présente invention, le support tubulaire peut être préparé par des méthodes telles que la lyophilisation, le moulage, l'extrusion, l'évaporation de solvant, l'extraction d'agents porogènes ; l'immersion-précipitation » ou une association de ces méthodes.

En accord avec la présente invention, la bioprothèse composée peut être utilisée pour la réparation, le remplacement ou la régénération d'organes creux humains incluant le tractus gastro intestinal, les conduits digestifs, biliaire, pancréatique, urinaires, et génitaux ainsi que les vaisseaux sanguins et tissus nerveux.

La bioprothèse complexe de la présente invention est constituée d'un support présentant des caractéristiques optimales pour une croissance ectopique de matériel biologique et de préférence de matériel foetal. Le support est une structure tubulaire biodégradable permettant la régénération d'organes creux tels que les conduits digestifs, biliaire, pancréatique, urinaires et génitaux (oesophage, intestin, estomac, cholédoque, urètre, uretère, vessie, trompes de Fallope et utérus). Le concept n'est pas de remplacer une partie défectueuse de manière permanente par le support tubulaire biodégradable mais de promouvoir/stimuler la régénération tissulaire par l'utilisation d'un support tubulaire biodégradable associé à un transplant de matériel biologique vivant de préférence peu ou pas différencié et de préférence d'origine foetal ou associé à au moins une partie des cellules du tissu de l'organe à reconstruire..

Le support est conçu comme un tube avec une couche externe poreuse qui peut stimuler la migration cellulaire/tissulaire, la vascularisation et la régénération d'organes creux. La lumière interne du tube n'est pas perméable et peut être en contact avec le bol alimentaire ou tout autre fluide circulant dans la partie creuse de l'organe. Il présente également des dimensions et une taille en accord avec l'organe à reconstituer.

Comme exposé précédemment, le support tubulaire qui sert de support à la matière biologique vivante peut être constitué de différents polymères, dès l'instant où ils pourront être mis en oeuvre de façon à obtenir un tube présentant des dimensions analogues à celles de l'organe ou de la partie d'organe à reconstituer et des propriétés mécaniques suffisantes (élasticité, résistance, flexibilité sans diminution de la forme et de la lumière) ainsi qu'une porosité adaptée pour assurer une bonne adhésion de la matière biologique, et de préférence de la matière foetale, au cours de sa croissance in vivo et assurer une circulation normale du fluide circulant normalement dans l'organe creux.

Plus précisément, la porosité doit être de taille suffisante pour permettre l'infiltration cellulaire et la colonisation par les vaisseaux sanguins ainsi que la croissance de la matière biologique et de préférence de la matière foetale.

Les pores sont de préférence interconnectés pour permettre les interactions cellulaires, la diffusion d'oxygène et de métabolites.

La porosité sera de préférence continue sur toute l'épaisseur du tube, jusqu'à sa surface interne.

Le diamètre interne doit être adapté à la taille du conduit à reconstruire. Le choix du diamètre externe est moins important. On doit toutefois tenir compte de la flexibilité du tube qui doit être conservée.

La surface ou couche interne du tube doit être imperméable et non poreuse, de façon à permettre l'étanchéité de ce tube au chyme dans la cas des conduits digestifs (par exemple l'oesophage et l'estomac), au gaz dans le cas de conduit respiratoire (par exemple la trachée) ou de tout autre fluide dans le cas des autres organes, de façon à éviter le passage des bactéries et des virus. D'autre part cette surface interne est constituée d'une couche essentiellement non poreuse empêchant sensiblement la prolifération cellulaire afin d'éviter une prolifération cellulaire non dirigée qui à terme fermerait la lumière du support tubulaire.

D'une façon générale, la résistance mécanique du tube est, de préférence, suffisante pour éviter l'écrasement du tube et maintenir la lumière (diamètre interne) assurant le passage de l'air ou du bol alimentaire ou de tout autre fluide en fonction de l'organe à reconstruire.

Le matériau polymère a, de préférence, une dégradation dans le temps nécessaire à la régénération de l'organe. Il doit en outre être biocompatible de façon à ne pas induire de toxicité cellulaire, de réaction inflammatoire ou de réaction de rejet et il devra être en outre compatible avec le matériel biologique, et de préférence de matériel foetal.

Par ailleurs, le matériau polymère doit pouvoir être aisément stérilisé.

Comme exposé précédemment, le support est avantageusement constitué de chitosane qui est un matériau facilement disponible et pouvant conduire par un procédé simple à tous les avantages exposés ci-dessus. Toutefois, un nombre important d'autres polymères connus pour leurs propriétés de biodégradabilité et de biocompatibilité pourront être choisis.

Plus précisément, le matériau polymère est choisi dans le groupe constitué du chitosane, de la chitine, d'un copolymère chitine-glucane, et de leurs dérivés ou copolymères, ces polymères étant associés éventuellement à au moins un autre polymère biocompatible et biodégradable.

Différents autres polymères biocompatibles et biodégradables pourront être utilisés en association avec le chitosane, la chitine ou leurs dérivés ou copolymères définis ci-dessus, notamment pour faire varier une ou plusieurs de leurs propriétés, tel que leur capacité de prolifération cellulaire, leur résistance mécanique, leur taux de gonflement au contact du milieu biologique de l'hôte avoisinant la prothèse, leur déformabilité, leur vitesse de dégradation, leur compressibilité, élasticité, souplesse, flexibilité, etc.

On pourra en particulier recourir à des biopolymères, en particulier des biopolymères choisis dans le groupe constitué des glycosaminoglycanes (GAG), en particulier le hyaluronate, le sulfate de chondroitine ou l'héparine, des collagènes, des alginates, des dextranes et de leurs mélanges.

On pourra également choisir des polymères de synthèse biodégradables et biocompatibles, en particulier choisis dans le groupe constitué des polyesters biodégradables de synthèse tels que les homopolymères et copolymères basés sur l'acide lactique, l'acide glycolique, l'epsilon-caprolactone et la p-dioxanone ou encore tout autre polyester naturel comme ceux de la famille des polyhydroxyalcanoates comme les homo- et copolymères basés sur l'hydroxybutyrate, l'hydroxyvalérate, les polyorthoesters, les polyuréthanes.

On utilisera de préférence le chitosane ou un matériau polymère en contenant.

Le chitosane est fabriqué par déacétylation de la chitine dont les différentes sources possibles sont bien connues. Il s'agit de la carapace des crustacées (crabes, crevettes et homards essentiellement), des endosquelettes, des céphalopodes, des cuticules, des arthropodes, des diatomées et des parois cellulaires des champignons. On choisira de préférence des polymères d'origine fongique, du fait de leur caractère hypoallergénique, de leur qualité constante et aisément traçable, de leur source quasiment illimitée et totalement renouvelable, permettant en outre une valorisation de sous-produits de l'industrie agroalimentaire et biotechnologique. On peut avantageusement produire le chitosane à partir du procédé décrit dans la demande de brevet WO 03068824 de KITOZYME.

Le chitosane a de préférence un degré de déacétylation et une masse moléculaire choisis de façon à assurer une vitesse de dégradation optimale. Il a par exemple été montré que la vitesse de dégradation du chitosane dépend fortement de sa masse moléculaire et de son degré de déacétylation, en ce sens que plus la masse moléculaire et le degré de déacétylation sont faibles, plus la dégradation est rapide. Aussi, le contrôle de la porosité est important, des supports avec des tailles de pores plus élevées et de porosités plus grandes se dégradent plus rapidement.

Le chitosane lorsqu'il sera choisi pour la préparation des tubes servant de support peut être associé à différents polymères biodégradables, par exemple un autre glycopolymère tel que la chitine ou la chitine-glucane. Des méthodes de préparation de ces polymères ou copolymères sont décrites dans les demandes de brevet de KITOZYME (WO 03068824, FR 05 07066 et FR 06 51415).

Comme exposé précédemment, la porosité du support tubulaire est essentielle pour permettre l'accrochage et la croissance de la matière biologique, et de préférence la matière foetale, après l'incorporation de la prothèse in vivo.

Cette porosité doit être suffisante pour laisser passer les cellules du sang au minimum, et éventuellement certaines cellules du greffon. Le diamètre des pores de la partie poreuse est donc supérieur à 10 µm et de préférence compris entre 10 et 200 µm.

Le diamètre interne et l'épaisseur du tube constituant le support sont adaptés à ceux de l'organe creux que l'on souhaite reconstruire.

Les dimensions, en particulier l'épaisseur du polymère dépendent des propriétés physiques visées, ces propriétés devant garantir une élasticité et une résistance en relation avec la nature de l'organe à reconstruire. Cette épaisseur dépend également du diamètre du tube et de la nature de l'organe à reconstruire. On comprend que, en tout état de cause, le diamètre intérieur du tube est donné par le diamètre de l'organe à reconstruire.

Le matériel vivant peut être placé à la surface, ou dans, la couche externe et poreuse du tube, et éventuellement maintenu en place par un tissé enroulé autour de ce dernier. Une autre possibilité est de placer le matériel vivant entre la surface interne imperméable du tube (couche essentiellement non poreuse) et sa surface poreuse. Dans ce dernier cas, la couche poreuse et la couche essentiellement non poreuse peuvent ne pas être solidaires et sont conçues de façon indépendante. Ils peuvent être donc physiquement indépendants. La couche non poreuse peut-être un film ou un second tube non poreux. On entend par essentiellement non poreuse, le fait que les cellules ou le matériau biologique associé au polymère biodégradable ne colonise pas totalement ou peu, et de préférence pas, la couche non poreuse.

L'ajout du matériel biologique sur ou dans le support tubulaire biodégradable se fait de préférence in vivo ou juste avant la résection.

Selon un mode de réalisation, le support tubulaire creux biodégradable est implanté pour remplacer au moins une partie d'un organe creux, puis le matériau d'origine biologique vivante est introduit à la surface de, ou dans, la couche poreuse, ou à la surface de la couche essentiellement non poreuse faisant face à la couche poreuse. La prolifération de la matière d'origine biologique vivante s'effectue donc in vivo. Ceci permet une reconstruction très avantageuse de l'organe creux ou partie d'organes creux à reconstruire ou remplacer.

Selon un second mode de réalisation, le matériel biologique est ajouté au support juste avant résection pour éviter une étape de culture du matériel biologique.

Selon un troisième mode de réalisation, le support tubulaire biodégradable est implanté sans matériel biologique. Le support est ensuite colonisé par les cellules hôtes.

Selon un quatrième mode de réalisation, le support tubulaire est réalisé en deux parties séparées et indépendantes physiquement (c'est à dire manipulables indépendamment), une première partie comprenant la couche poreuse, et la seconde partie comprenant la couche essentiellement non poreuse. Dans ce cadre, la couche non poreuse est placée à l'intérieur de l'organe tubulaire creux à reconstruire puis la couche poreuse est placée à l'extérieur de cet organe.

Ces modes de réalisation permettent notamment de s'affranchir des conditions in vitro d'ensemencement et de culture cellulaire, mais aussi permet un gain de temps et des coûts de production. D'autre part il n'est pas nécessaire de constituer une banque cellulaire. Dans ces modes avantageux de réalisation, la prothèse est destinée à une incorporation in vivo de la matière d'origine biologique vivante. La colonisation cellulaire effectuée in vivo est très bonne et la reconstruction de la partie ou totalité d'organe remplacée est permise.

Le matériel biologique d'origine humaine peut être d'origine cellulaire (exclus les cellules souches embryonnaires) et de préférence des cellules souches germinales, en ce compris les cellules prélevées sur le foetus de plus de 8 semaines, en particulier sur le foetus entre 8-10 semaines, ou au sein du cordon ombilical après la naissance. De préférence le matériel vivant utilisé est peu ou pas différencié et, de préférence, d'origine foetale. Il peut aussi être constitué par les cellules prolifératives du tissu à reconstruire.

Les cellules souches foetales (prélevées sur le foetus de 8-10 semaines) seront utilisées de manières préférentielles par rapport aux cellules souches adultes car plus abondantes. Les cellules souches adultes, seront de préférences prélevées dans l'organe à reconstruire (estomac, intestin, utérus, vessie, vaisseaux sanguins).

Les cellules peuvent être des cellules d'au moins un animal, notamment d'un mammifère, ou d'au moins un être humain.

Le matériel foetal peut être soit un organe, soit un segment d'organe, soit une émulsion de cellules. Ce matériel foetal est avantageusement sous une forme humide et visqueuse, de façon à pouvoir être étendu sur une surface, celle du tube à laquelle il devra adhérer ou à laquelle il devra être fixé, formant une sorte de pansement en réseau. Une autre alternative consiste en l'utilisation de cellules souches dont la différentiation serait contrôlable.

L'épaisseur de la couche déposée sera avantageusement de 0,1 à 1 mm mais pourra également être supérieure. L'homme du métier comprend que l'épaisseur de cette couche dépend essentiellement de la nature de l'organe et de la nature de son receveur (humain ou animal).

Les proportions de polymère et de matériel biologique, et de préférence de matière foetale, peuvent également varier dans de larges proportions en fonction de la nature de l'organe à reconstruire

Les avantages de l'utilisation de matériaux foetaux sont :
- Un haut degré de survie du transplant même en l'absence de vascularisation (grâce à la diffusion des nutriments en attendant la colonisation par les vaisseaux de l'hôte)

Les organes foetaux sont suffisamment non différenciés pour permettre une capacité élevée de croissance et de régénération d'organes matures tout en étant suffisamment différenciés pour éviter toute erreur dans leur développement et leur croissance (pas de développement déviant observé à partir de matériel foetal d'origine). La différenciation des matériaux foetaux est plus aisée et permet par exemple un meilleur contrôle de la différenciation que lors de l'utilisation des cellules souches
- Le matériel foetal ne contient pas d'agents infectieux et réduit donc les risques de transmissions virales.

Comme exposé précédemment, l'invention comprend également le procédé de préparation de la prothèse de l'invention.

Ce procédé comprend la préparation d'un support tubulaire poreux comprenant une couche essentiellement non poreuse sur sa face interne et l'incorporation à la surface de ce support tubulaire et/ou en son sein d'un matériau d'origine foetale.

Comme exposé précédemment, on connaît déjà des supports tubulaires poreux, et notamment des supports à base de chitosane. Ces supports peuvent être utilisés pour la préparation des prothèses de l'invention.

D'une façon générale, la technique de fabrication de tubes à base de polymère présentant une structure poreuse et une couche interne non poreuse (imperméable) est bien connue.

La lyophilisation est une méthode bien connue pour la préparation de matériaux poreux. Son principe repose sur la congélation d'une solution afin d'induire la cristallisation du solvant.

Le solvant est ensuite éliminé par sublimation sous vide afin de créer des pores en lieu et place des cristaux de solvant. Cette technique combine les avantages suivants :
Simplicité de mise en oeuvre
   - Possibilité de contrôler la porosité et le diamètre des pores en jouant sur les paramètres de mise en oeuvre et de formulation (vitesse de refroidissement, concentration de la solution polymère,...)
   - Différentes types de géométries sont accessibles : membranes poreuses, supports 3D, billes, tubes
   - Extrapolation industrielle facilement envisageable.

Comme décrit dans la publication : « Porous chitosan scaffolds for tissue engineering » (S.V. Madihally, H.W.T. Matthew, Biomaterials 20 (1999), 1133-1142), des tubes poreux en chitosane sont préparés par lyophilisation, en congelant une solution de chitosane dans l'espace annulaire compris entre 2 tubes concentriques (silicone ou polytétrafluoroethylene), la solution de chitosane est injectée dans cet espace et l'ensemble est congelé par contact direct soit avec la carboglace à -78°C (comme décrit dans l'article) Le tube externe est ensuite retiré et l'ensemble est lyophilisé. Réalisé selon cette méthode, le tube est complètement poreux sur toute son épaisseur, y compris la surface externe et la surface interne (ou surface luminale).

Pour obtenir des tubes caractérisés par une paroi luminale non poreuse, différentes solutions pourront être utilisées, les mêmes auteurs décrivent une méthode basée sur le recouvrement préalable du tube interne en silicone par un film de chitosane. Ce film de chitosane peut-être obtenu en trempant le tube dans une solution de chitosane et en le gélifiant par immersion rapide dans une solution d'ammoniaque à 30% et en le laissant ensuite sécher. On pourra également préparer le film directement par simple évaporation du solvant, comme cela est réalisé dans l'exemple 1, ce qui est plus avantageux.

Une fois réhydratés en milieu aqueux, les supports décrits ci-dessus vont rapidement gonfler et finir par se re-dissoudre, du fait de la présence d'acétate de chitosane soluble au sein de la structure lyophilisée. Par ailleurs, la dissolution des supports peut être évitée en neutralisant les échantillons par immersion soit dans une solution de NaOH, soit dans une série d'alcools de concentration décroissante (S.V. Madihally, H.W.T. Matthew, Biomaterials 20 (1999), 1133-1142).

On utilisera avantageusement des concentrations en chitosane entre 1 et 10% dans l'acide acétique pour la préparation des tubes poreux par lyophilisation.

En dehors de la technique de lyophilisation ou séparation de phase induite thermiquement, d'autres techniques destinées à former des pores sont bien connues pour la préparation de supports poreux.

On citera : l'extraction de sels porogènes, le moussage en fluide supercritique (CO2 supercritique), ainsi que des méthodes plus récentes comme le modelage d'émulsion, la technique connue sous le vocable anglais de « solid free-forming » qui consiste à construire des contours d'objets tridimensionnels mais la plupart de ces méthodes ne permettent pas un bon contrôle de la porosité et génère des structures poreuses faiblement connectées.

Pour rendre la lumière du tube non poreuse, un tube non poreux peut être inséré à l'intérieur du tube poreux ou encore, le tube non-poreux creux peut être entouré par une membrane poreuse constituant la partie externe poreuse du support tubulaire. Dans ces cas, les couches poreuse et non-poreuse peuvent être alors physiquement indépendantes. Dans cette variante, on peut prévoir un bourrelet situé à chaque extrémité du tube non poreux, de manière à améliorer l'étanchéité de la jonction tube non poreux-oesophage. Ce bourrelet peut être réalisé au moyen de fils disposé préalablement sur le tube non poreux, par une surépaisseur du matériau du tube ou d'un matériau différent du tube non poreux. Ce bourrelet peut également faciliter la solidarisation du tube à l'oesophage.

Un tube poreux de chitosane présentant une résistance mécanique suffisante est obtenu par lyophilisation de solutions de chitosane. Les solvants utilisés pour dissoudre le chitosane sont des acides organiques et inorganiques tels que les acides formique, lactique, succinique, l'acide chlorhydrique, l'acide gluconique et de manière préférentielle l'acide acétique. Ils peuvent être utilisés pour confectionner les tubes de chitosane.

Idéalement, les solutions de chitosane sont préparées par dissolution de chitosane à des concentrations de 1-10% dans une solution aqueuse d'acide acétique.

Idéalement, le chitosane utilisé comme matériel de départ pour la conception de bioprothèses est de nature fongique et obtenu par déacétylation de la chitine extraite de champignons, par exemple selon les procédés décrits dans les demandes de brevets de Kitozyme indiquées plus haut.

Le chitosane présente avantageusement un degré d'acétylation et une masse moléculaire choisis de façon à obtenir une vitesse de dégradation optimale et en adéquation avec la vitesse de régénération de l'organe à régénérer.

La neutralisation du support de chitosane est avantageusement obtenue par un traitement par la soude afin d'obtenir un support compatible avec les conditions physiologiques. Il est préférable de traiter avec une solution de NaOH à 1%.

Le support de chitosane peut être stérilisé par des méthodes d'irradiations- y ou éthylène oxyde, ou par autoclavage.

La présente invention couvre un support tubulaire biodégradable, tel que défini précédemment, destiné à la reconstruction d'au moins une partie d'un organe creux.

La présente invention couvre aussi un matériaux polymère biocompatible et biodégradable poreux pour la chirurgie réparatrice d'un organe creux de forme tubulaire, ledit matériaux polymère étant destiné à former la couche poreuse d'un support tubulaire creux biodégradable comprenant ou constitué d'une couche externe poreuse et d'une couche interne essentiellement non poreuse.

Avantageusement, le support tubulaire creux biodégradable est positionné in fine de manière à ce que la couche externe poreuse soit positionnée sur la surface extérieure de l'organe creux, et la couche interne essentiellement non poreuse soit positionnée sur la surface intérieure de l'organe creux.

Selon un mode de réalisation, le matériau polymère de forme tubulaire comprend une extrémité distale et une extrémité proximale, ladite extrémité proximale étant destinée à être positionnée à une extrémité d'un organe creux sectionné en totalité ou partiellement, ladite extrémité distale étant destinée à être positionnée à une autre extrémité de l'organe creux sectionné en totalité ou partiellement.

Cette disposition permet de remplacer ou reconstruire une section totale ou partielle de l'organe creux.

On comprend aisément que le terme extrémité de l'organe creux s'entend au sens large et concerne dans le cas d'une section partielle d'un organe creux une partie du tissu de l'organe faisant face à une autre partie du tissu pouvant être reliées géométriquement par une droite traversant la partie sectionnée du tissu, ladite droite ne traversant pas le lumen de l'organe creux.

La présente invention couvre une méthode de prolifération cellulaire, notamment pour reconstruire au moins une partie d'un organe creux, les étapes comprenant la réalisation d'un support tubulaire biodégradable comprenant une couche externe poreuse et une couche interne essentiellement non poreuse, et l'ensemencement de cellules ou de l'implant tissulaire à la surface externe, et/ou ou au sein d'au moins une partie de la couche poreuse dudit support, et/ou sur la surface de la couche essentiellement non poreuse faisant face à la couche poreuse, dans des conditions permettant leur prolifération au sein de la couche poreuse.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

### EXEMPLES

### Exemple 1 Fabrication de tubes poreux de chitosane

Un chitosane d'origine végétale produit par KitoZyme, caractérisé par sa masse moléculaire viscosimétrique de 42 K et un degré d'acétylation de 11% est mis en solution dans l'acide acétique (1%) à raison de 5% (poids/volume).

Le support poreux de forme tubulaire est fabriqué par lyophilisation de la solution de chitosane, préalablement injectée à l'aide d'une seringue dans l'espace annulaire formé par 2 tubes concentriques de diamètres différents. L'ensemble est congelé par contact direct dans l'azote liquide pendant 15 minutes. Le tube externe est ensuite retiré et on poursuit la lyophilisation de l'ensemble pendant 24h. Après séchage, le tube interne est à son tour retiré et le tube obtenu est analysé par microscopie électronique à balayage.

Les figures 2A, 2B, 2C et 2D représentent les clichés obtenus et illustrent la structure du tube. La figure 2A est une coupe en section transversale qui montre bien que la porosité est obtenue sur l'ensemble de l'épaisseur du tube. La figure 2B montre particulièrement bien cette structure poreuse. La surface interne du tube est illustrée par la figure 2C qui montre que les pores ne s'ouvrent pas sur la lumière du tube. La figure 2D qui donne l'aspect de la couche externe du tube montre que les pores au contraire s'ouvrent très clairement vers l'extérieur du tube.

### Exemple 2: Histologie après implantation sous-cutanée des membranes et de tubes poreux chez le rat et la souris

Des supports poreux de chitosane sous forme de tubes, préparés par lyophilisation de solutions de chitosane dans l'acide acétique ont été d'abord neutralisés par traitement avec une solution de NaOH (pour éliminer les résidus d'acide) puis stérilisés soit par exposition pendant 20 min dans l'alcool à 96° suivi d'un lavage dans un tampon salin pendant 5 min, soit par autoclavage. 10 souris BALBc et 5 rats Fisher ont reçu les implants de chitosane (membranes ou tubes), en sous-cutané, un dans chaque oreille.

Des tubes en paraffine et polyéthylène ont été utilisés comme contrôle.

A différents intervalles de temps (7, 14 et 62 jours), des analyses biométriques externes et des analyses histologiques ont été réalisées.

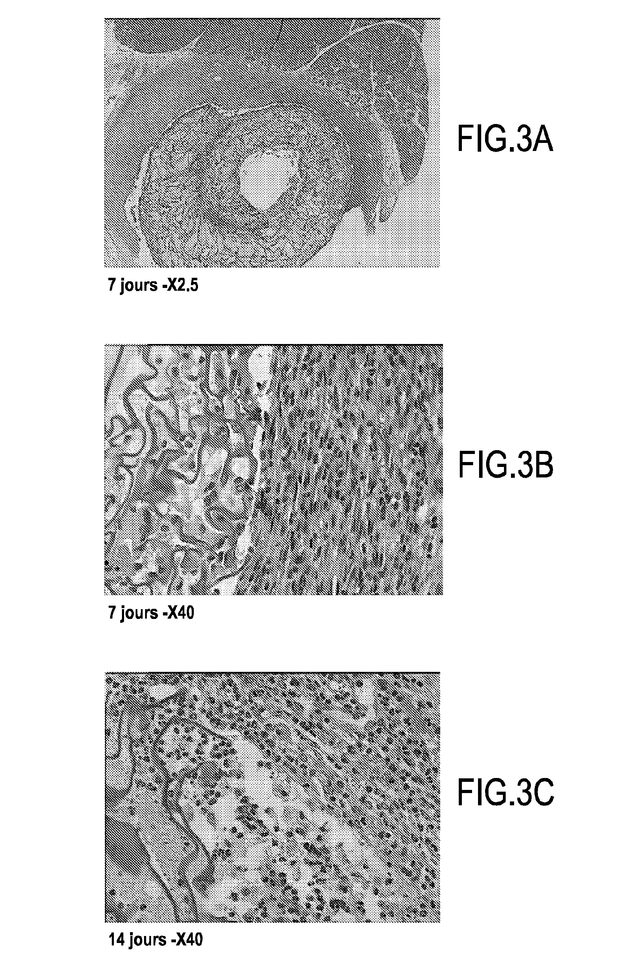

Les résultats montrent que dans tous les animaux, les implants de chitosane sont bien tolérés (figure 3A) et infiltrés par les cellules et tissus environnants déjà après 7 jours (figure 3B). Une réaction inflammatoire modérée est observée et aucun n'implant n'a induit de réaction de rejet. Les implants en chitosane commencent à se dégrader entre les semaines 1 à 4 (figure 3C) et sont complètement résorbés après 62 jours.

En conclusion de cet exemple, les prothèses de chitosane sont biocompatibles, permettent l'infiltration de cellules et tissus avoisinants et n'entraînent qu'une réaction inflammatoire très limitée au cours de leur dégradation.

### Exemple 3: Implantation sous-cutanée des membranes et de tubes poreux associé à du matériel foetal chez la souris

De l'intestin foetal a été prélevé chez des foetus de souris après 15 à 20 jours de développement intra-utérin et implanté dans des poches sous-cutanées réalisées dans le pavillon oculaire de souris hôtes (10 souris) ; ce matériel foetal étant associé à un implant de chitosane sous forme de tubes juste avant implantation par recouvrement de la surface externe du support tubulaire de chitosane par la matière foetale. La souche du donneur et du receveur est identique (transplantation syngénique) pour éviter des réactions de rejets immuno-biologiques.

Dans cet exemple, les tubes de chitosane ont été stérilisés par traitement à l'alcool pendant 30 à 40 minutes avant d'être lavés avec une solution saline stérile pendant 5 puis 25 minutes afin d'éliminer tout résidu d'alcool.

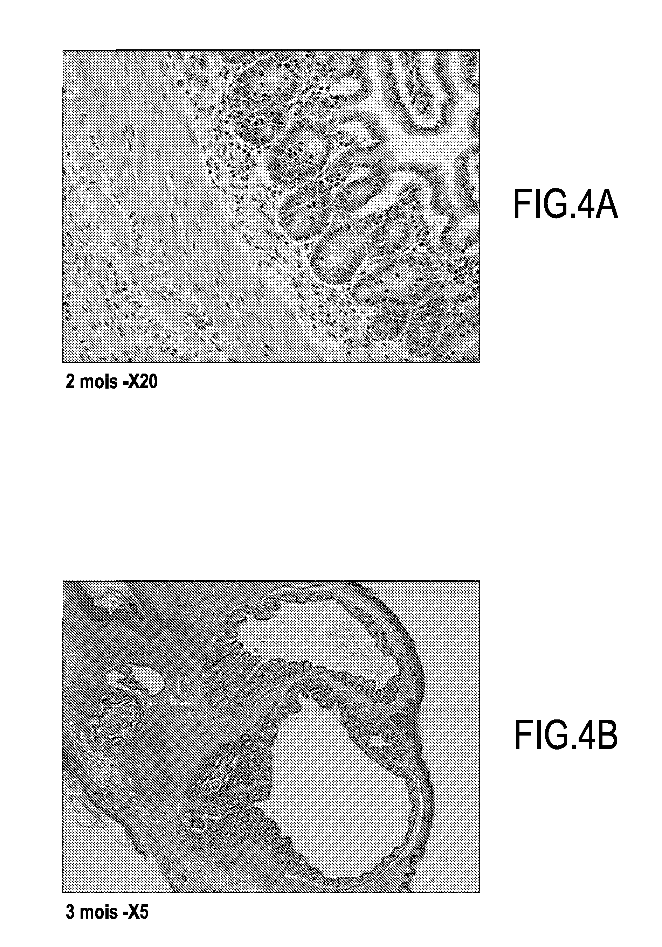

Après 2 (figure 4A) et 3 mois (figure 4B), les implants d'intestins montrent un développement excellent, le support de chitosane étant complètement dégradé au bout de ce délai. Après 2 mois (figure 4A), une coupe transversale au niveau de l'implant foetal montre le développement d'un intestin normal semblable à de l'intestin adulte et présentant toutes ses caractéristiques (structure de villosités) en présence du tube de chitosane qui, lui, est complètement résorbé. Cette expérience montre donc que les implants de chitosane sont compatibles avec le développement de transplants foetaux syngéniques d'organes digestifs, dans ce cas, l'intestin. La lumière intestinale est apparente sur la figure 4B.

Des coupes histologiques réalisées au niveau des poumons, du foie et des reins chez l'hôte à des délais de 3 mois démontrent l'absence de réaction inflammatoire et d'effet nocif au niveau de ces organes.

### Exemple 4 : Simulation d'une bioprothèse d'oesophage

Des prothèses combinées composées de tube poreux de chitosane et de matériel intestinal foetal (le matériel foetal étant placé soit à l'extérieur du tube, soit entre la couche poreuse et la surface interne non poreuse du tube) ont été implantées longitudinalement entre les muscles du cou chez le rat sans perturber l'oesophage. Cette expérience montre la capacité du tube de chitosane à être colonisé par du matériel intestinal foetal et à supporter les mouvements du cou.

### Exemple 5 : Remplacement d'un segment d'oesophage par l'utilisation d'un tube poreux de chitosane couvert par de l'intestin ou de l'oesophaae foetal syngénique

Des segments d'intestin foetal ont été collectés entre 14 et 18 jours de développement intra-utérin chez le rat et disposés autour des tubes poreux de chitosane. Après résection d'un segment d'oesophage de 0,5 à 1 cm de long au niveau du cou du rat, le tube de chitosane avec le matériel foetal est fixé aux deux extrémités sectionnées de l'oesophage du rat de telle façon que les jonctions entre la prothèse et l'organe soient hermétiques ou étanches. La même expérience est répétée avec du matériel d'oesophage foetal.

### Exemple 6 : Tolérance après implantation au niveau du cou du rat d'un support tubulaire creux de chitosane

Le support tubulaire creux de l'exemple 6 est composé d'un premier tube non-poreux préparé selon le procédé décrit dans WO2007042281 à partir d'un échantillon de chitosane de l'Exemple 1, et caractérisé par un diamètre interne de 1.5mm et un diamètre externe de 2.5 mm. Le tube non-poreux est entouré d'une membrane poreuse préparée selon un procédé classique de lyophilisation à partir de chitosane de l'Exemple 1 constituant ainsi la couche externe poreuse du support tubulaire. Le tube non-poreux et la membrane sont tous deux stérilisés (autoclavage ou immersion dans une solution alcoolisée désinfectante pendant 15 à 20 minutes) puis rincés dans une solution physiologique (NaCl 0.9%) pendant au moins 20 minutes.

Le rat anesthésié est placé sur un support adéquat sur le dos, en extension de façon à présenter la face antérieure du cou. Une section médiane de la peau depuis le niveau du cartilage thyroïde jusqu'à celui de la fourchette sternale est pratiquée, les muscles sous cutanés sont incisés, les muscles pré-trachéaux fendus longitudinalement et dans l'interstice le tube de chitosane entouré de la membrane poreuse de chitosane est placé longitudinalement. Les plans musculaire et sous cutanés sont refermés par suturation.

Après sacrifice au jour 90 (3 mois), l'animal ne présentait aucune altération macroscopique de l'aspect extérieur, des organes internes. L'étude anatomo-pathologique a montré une disparition presque complète de la membrane, une conservation du tube, bien entouré de tissus fibreux, pas d'altération macroscopique ni microscopique des organes internes et des tissus environnants le tube et la membrane.

### Exemple 7 : Implantation d'un support tubulaire creux au niveau d'un oesophage partiellement sectionné

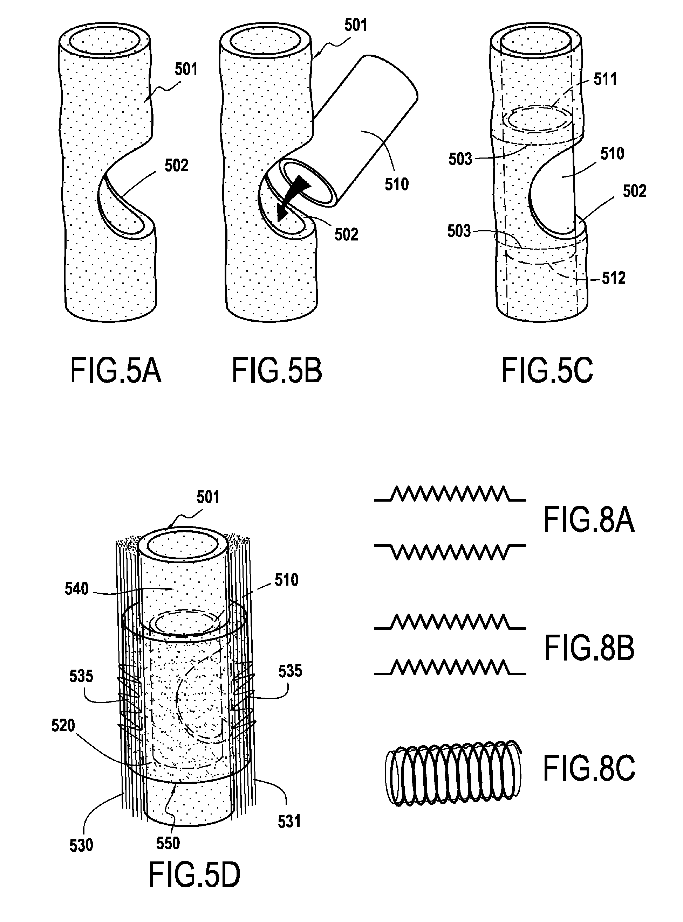

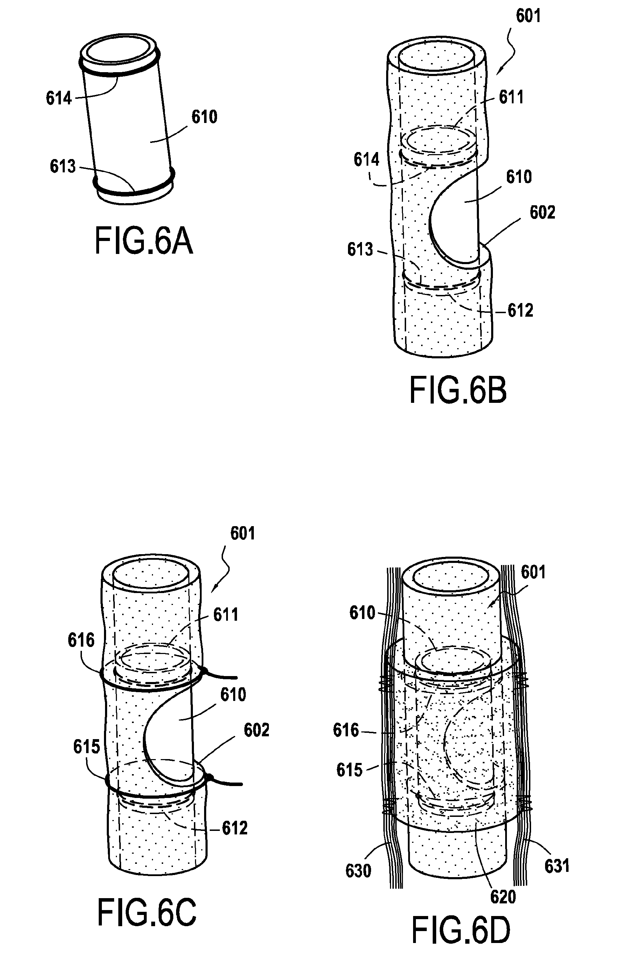

Les figures 5 et 6 servent à supporter schématiquement cet exemple. Elles ne constituent en aucun cas une représentation de la réalité du détail et des proportions qui ne sont pas respectées.

Un rat anesthésié a subi une section longitudinale de la peau du cou suivie de la mise en évidence de la trachée et de l'oesophage (501) dont une partie (502) (environ 2/3 du pourtour) a été sectionnée (Fig. 5A). Le tube non-poreux (510) de l'Exemple 6 est ensuite introduit à l'intérieur de l'oesophage (501) via la partie de l'organe sectionné (502)(Fig. 5B) puis fixé à l'aide de fils (503) préalablement placés autour de l'oesophage (501) et serrant l'ensemble tube+oesophage au niveau des extrémités du tube (511, 512)(Fig. 5C). La membrane poreuse (520) de l'Exemple 6 est ensuite enroulée autour de l'oesophage+tube puis fixée aux tissus musculaires adjacents (530, 531) au moyen d'un point de suture (535) (Fig. 5D). Le matériau vivant (540) est donc placé in situ entre la face externe poreuse (520) et la face interne non-poreuse (510) du support tubulaire (550).

Le décours post opératoire est sans complication locale (déhiscence des sutures, abcès, infection superficielle). L'animal éprouve quelques difficultés à boire et s'alimenter durant 10 jours et perd du poids, puis la situation s'améliore rapidement. Le rat est sacrifié après 35 jours. L'observation anatomo-pathologique révèle qu'après 35 jours le rat a retrouvé son poids initial. Les organes internes ont un aspect normal.

**L'**oe**sophage est étanche, non rétréci et reconstitué.** Aucun abcès local ni fuite (du bol alimentaire, liquide corporel, etc) n'a été observé. La prothèse a donc permis de reconstituer une liaison étanche avec l'oesophage.

L'analyse des coupes histologiques montre que la prothèse a bien disparu du site (elle n'a été retrouvée dans aucune partie du tube digestif, donc a été résorbée ou digérée), que l'oesophage et les tissus avoisinants ont un aspect normal. Quelques restes de membrane dans une zone légèrement infiltrée par la matière vivante ont été retrouvés dans le voisinage de l'oesophage cervical.

Exemple d'un mode opératoire possible pour l'implantation des matériaux :
1. Préparation du tube de chitosane non poreux (610) et de la membrane poreuse (620) après leur stérilisation soit par autoclave soit par immersion dans une solution alcoolisée désinfectante durant 15 à 20 min :
   ► rincer le tube (610) au moyen d'une solution physiologique stérile pendant 20 min au moins ;
   ► rincer la membrane poreuse (620) avec la même solution physiologique (NaCl 0.9%) durant le même temps ;
   ► Placer 2 fils de suture de repère et de raccord (613, 614) formant une sorte de bourrelet autour des deux extrémités (611, 612) du tube (610) (Figure 6A) Ce bourrelet forme une surépaisseur au niveau des extrémités du tube (610) et peut également faciliter la solidarisation du tube à l'oesophage.
2. Mise en évidence de la trachée et de l'oesophage (601), dissection de l'oesophage,
3. Section partielle de l'oesophage (601) (section sur environ les 2/3 du pourtour) avec ou sans ablation faisant apparaître une cavité (602)
4. Insertion d'une extrémité du tube (610), et fixation dans l'oesophage (601) au moyen des fils (615,616) entourant chaque bord libre de l'oesophage (601) sectionné de telle sorte que le bourrelet formé par le fil (613,614) soit à l'intérieur de l'organe, insertion et fixation de l'autre extrémité de façon analogue. (figures 6B et 6C)
5. Enroulement de la membrane poreuse (620) (stérilisée et rincée de la même façon que le tube) à l'extérieur de l'oesophage (601) et du tube (610) (au niveau de leur interface). Un point de suture est placé entre les bouts de la membrane (620) et les tissus adjacents (630, 631) afin de fixer la membrane (620) (figure 6D). Les fils 615 et 616 peuvent également être utilisés pour fixer la membrane poreuse (620).
6. Fermeture de la plaie opératoire par plans.

### Exemple 8 : Implantation d'un support tubulaire creux au niveau d'un oesophage complètement sectionné

Une section longitudinale de la peau du cou suivie de la mise en évidence de la trachée et de l'oesophage d'un rat anesthésié a été réalisée. L'oesophage cervical est complètement sectionné à mi-hauteur. Le tube non-poreux de l'Exemple 6 est ensuite introduit à l'intérieur de l'oesophage puis fixé à l'aide de fils préalablement positionnés autour de chaque bord libre de l'oesophage sectionné pour serrer l'ensemble tube+oesophage au niveau des extrémités du tube et positionner le tube de manière à reconstruire le tube formé par l'oesophage. La membrane poreuse de l'Exemple 6 est ensuite enroulée autour de l'oesophage et du tube puis fixée aux tissus musculaires adjacents au moyen d'un point de suture. Le matériau vivant est donc placé entre la face externe poreuse et la face interne non-poreuse du support tubulaire.

Le décours post opératoire est sans complication locale (déhiscence des sutures, abcès, infection superficielle). L'animal éprouve quelques difficultés à boire et s'alimenter.

Les observations ont été réalisées 1, 3 et 6 jours après l'intervention. La jonction tube-oesophage était étanche. Aucune infection ni abcès locaux n'ont été observés. Les coupes histologiques montrent une petite réaction inflammatoire locale et confirment la présence du tube et de fragments de membrane.

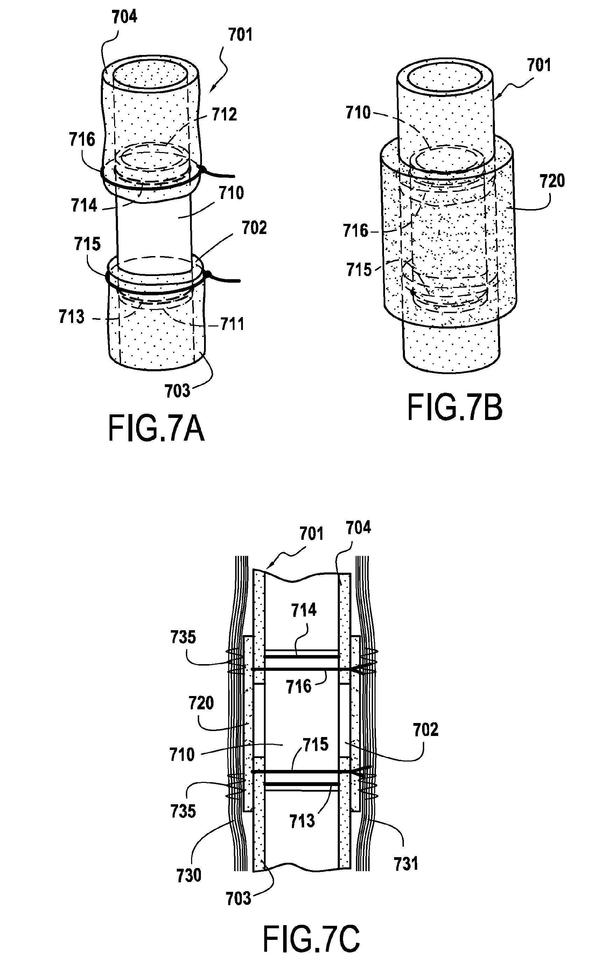

Exemple d'un mode opératoire possible pour l'implantation des matériaux :
Les premières étapes opératoires réalisées pour l'Exemple 8 sont identiques aux étapes 1 et 2 de l'Exemple 7. Les étapes 3 et 4 diffèrent par le fait que l'oesophage subit une section non plus partielle mais bien totale. Elles sont décrites comme suit :
   3. Section complète de l'oesophage avec ou sans ablation (701) cervical à mi hauteur, créant ainsi une section totale (702).
   4. Insertion d'une extrémité (711) du tube non poreux (710), et fixation dans la partie distale (703) de l'oesophage (701) au moyen des fils (715, 716) entourant chaque bord libre (703, 704) de l'oesophage (701) sectionné, de telle sorte que le bourrelet formé par le fil (713, 714) sur le tube soit à l'intérieur de l'organe (701), insertion et fixation de l'autre extrémité (712) dans le segment proximal (704) de façon analogue. (Fig. 7A)
   5. Enroulement de la membrane poreuse (720) à l'extérieur de l'oesophage (701) et du tube (710) (Fig. 7B).
   6. Un ou plusieurs points de suture ou de colle (735) sont placés entre les bouts de la membrane et les tissus adjacents (730) afin de fixer la membrane (720) (Fig. 7C).
   7. Suture de la plaie en 2 plans - musculaire et cutané.

### Exemple 9 : Variante de réalisation du support tubulaire creux de l'invention

Un support tubulaire selon la présente invention réalisé conformément aux exemples précédents peut comporter une partie présentant des variations séquentielles de section de manière à former une structure du type accordéon permettant d'améliorer la flexibilité du tube, et donc la résistance au mouvement de l'hôte, et une déglutition plus. On peut par exemple mettre en oeuvre le protocole de l'exemple1 en utilisant deux espaces annulaires concentriques ayant chacun une partie comprenant des variations séquentielles de section à la place de l'espace annulaire formé par deux tubes concentriques de diamètres différents. On peut aussi mettre en oeuvre le protocole de préparation du tube non-poreux de l'exemple 6 pour préparer un tube non-poreux ayant une structure notamment de type accordéon permettant d'améliorer la flexibilité du tube.

Les figures 8A et 8B représentent schématiquement deux variantes de cette partie de tube.

De même, les espaces annulaires concentriques peuvent présenter différentes formes, comme par exemple pour présenter des variations d'épaisseur en forme de spirale. La figure 8C représente schématiquement une variante d'une spirale.

### Exemple 10 : Greffe d'intestin foetal ou d'oesophage seul

Etape 1 : (4 rats) Préparation des animaux comme dans l'exemple 6 mais le tube est remplacé par un segment d'intestin prélevé sur des foetus de rat de même souche âgés de 17 jours.

Etape 2 (1-2 mois plus tard) : Le cou est réouvert, le kyste formé par l'intestin ou oesophage foetal en croissance est ouvert et rincé, façonné de sorte à former un tube longitudinal de dimensions égales à celles de l'oesophage sans altérer ses liens vasculaires avec l'organisme, l'oesophage du receveur est mis en évidence, un segment est réséqué après pose de fils repères sur les bords de la section de chaque côté, puis le tube « foetal » est suturé à chaque extrémité de l'oesophage par un surjet 6.0. Si possible de la colle biologique est placée sur les sutures pour en renforcer l'étanchéité. Fermeture de la plaie opératoire en 2 plans.

Cet exemple montre la faisabilité d'une greffe de matière vivante d'origine foetale pour la reconstruction d'un organe tubulaire.

### Exemple 11- Plastie de segments oesophagiens avec combinaison tube + greffe

Méthode : L'exemple 10 est reproduit, mais un tube non-poreux en chitosane (préparé selon WO2007/042281) est fixé à l'intérieur du tube « foetal » afin de lui donner plus de rigidité et de résistance, le temps pour les sutures de se consolider et pour l'organisme de « réparer » le défaut circulaire oesophagien. Le tube peut ensuite être éliminé.

## Revendications

1. Prothèse destinée à promouvoir la reconstruction in vivo d'un organe creux ou d'une partie d'un organe creux, **caractérisée en ce qu'**elle comprend :
- un support tubulaire creux biodégradable comprenant au moins un matériau polymère biocompatible et biodégradable, ledit support étant constitué d'une couche externe poreuse et d'une couche interne essentiellement non poreuse et,
- un matériau d'origine biologique vivante à la surface externe, et/ou au sein d'au moins une partie de la couche poreuse dudit support, et/ou sur la surface de la couche essentiellement non poreuse faisant face à la couche poreuse, ledit matériau d'origine biologique étant choisi pour permettre la reconstruction in vivo dudit organe ou de ladite partie d'organe, ledit matériau d'origine biologique ne provenant pas d'embryon(s) humain(s).

2. Prothèse selon la revendication 1, **caractérisée en ce que** ledit matériau polymère est choisi dans le groupe constitué du chitosane, de la chitine, et de leurs dérivés ou copolymères, en particulier la chitine-glucane, ces polymères étant associés éventuellement à au moins un autre polymère biocompatible et biodégradable.

3. Prothèse selon la revendication 2, **caractérisée en ce que** ledit au moins un autre polymère biocompatible et biodégradable est un biopolymère choisi dans le groupe constitué des glycosaminoglycanes (GAG), en particulier le hyaluronate, le sulfate de chondroitine ou l'héparine, des collagènes, des alginates, des dextranes et de leurs mélanges.

4. Prothèse selon la revendication 2, **caractérisée en ce que** ledit au moins un autre polymère biocompatible et biodégradable est un polymère synthétique biocompatible et biodégradable choisi dans le groupe constitué des polyesters biodégradables de synthèse tels que les homopolymères et copolymères basés sur l'acide lactique, l'acide glycolique, l'epsilon-caprolactone et la p-dioxanone ou encore tout autre polyester naturel comme ceux de la famille des polyhydroxyalcanoate comme les homo- et copolymères basés sur l'hydroxybutyrate, l'hydroxyvalérate, les polyorthoesters, les polyuréthanes.

5. Prothèse selon l'une des revendications 1 à 4, **caractérisée en ce que** le diamètre des pores de la partie poreuse est supérieur à 10 µm, de préférence compris entre 10 et 200 µm.

6. Prothèse selon l'une des revendications 1 à 5, **caractérisée en ce que** ledit organe creux est un conduit digestif, biliaire, pancréatique, urinaire, génital ou sanguin, en particulier l'oesophage, l'intestin, l'estomac, le cholédoque, le conduit pancréatique, l'urètre, l'uretère, la vessie, les trompes de Fallope, l'utérus et les vaisseaux sanguins.

7. Prothèse selon la revendication 6, **caractérisée en ce que** ledit organe creux est l'oesophage.

8. Prothèse selon l'une des revendications 1 à 7, **caractérisée en ce que** ledit matériau d'origine biologique est un matériau d'origine foetale.

9. Prothèse selon la revendication 8, **caractérisée en ce que** ledit matériau d'origine foetale est un organe, un segment d'organe ou une émulsion de cellules d'origine foetale.

10. Prothèse selon la revendication 9, **caractérisée en ce que** ledit matériau d'origine foetale est sous une forme humide et visqueuse, de façon à améliorer son adhésion à la surface et/ou au sein dudit support.

11. Procédé de fabrication d'une prothèse selon l'une des revendications 1 à 10, **caractérisé en ce qu'**il comprend la préparation d'un support tubulaire biodégradable comprenant une couche externe poreuse permettant une prolifération cellulaire, ladite couche externe poreuse comprenant au moins un matériau polymère biocompatible et biodégradable, et une couche interne essentiellement non poreuse, et l'incorporation d'un matériel biologique destiné à former prothèse à la surface externe, et/ou au sein d'au moins une partie de la couche poreuse dudit support, et/ou sur la surface de la couche essentiellement non poreuse faisant face à la couche poreuse, ledit matériau d'origine biologique ne provenant pas d'embryon(s) humain(s)

12. Procédé selon la revendication 11, **caractérisé en ce que** la préparation de la couche poreuse externe, est effectuée par lyophilisation.

13. Support tubulaire biodégradable tel que défini à l'une quelconque des revendications 1 à 7, pour la reconstruction d'au moins une partie d'un organe creux.

14. Support tubulaire biodégradable selon la revendication 13, **caractérisé en ce que** le matériau polymère est choisi dans le groupe constitué du chitosane, de la chitine, et de leurs dérivés ou copolyères, en particulier la chitine-glucane, ces polymères étant associés éventuellement à au moins un autre polymère biocompatible et biodégradable.

15. Support tubulaire biodégradable selon la revendication 13 ou 14, **caractérisé en ce que** le diamètre des pores de la partie poreuse est supérieur à 10 µm, de préférence compris entre 10 et 200 µm

16. Support tubulaire biodégradables selon l'une des revendications 13 à 15, **caractérisé en ce que** ledit organe creux est un conduit digestif, biliaire, pancréatique, urinaire, génital ou sanguin, en particulier l'oesophage, l'intestin, l'estomac, le cholédoque, le conduit pancréatique, l'urètre, l'uretère, la vessie, les trompes de Fallope, l'utérus et les vaisseaux sanguins.

17. Support tubulaire biodégradable selon l'une des revendications 13 à 16, **caractérisée en ce que** ledit organe creux est l'oesophage.

18. Utilisation d'un support tubulaire selon l'une quelconque des revendications 13 à 17, pour la fabrication d'une prothèse pour la régénération d'un organe creux.

19. Utilisation, selon la revendication 18, pour la régénération de parties d'oesophage présentant une pathologie.

20. Utilisation, selon l'une quelconque des revendications 18 à 19, **caractérisée en ce que** l'organe creux comprend une partie atteinte d'une la pathologie quelconque ou d'un cancer.

## Patentansprüche

1. Prothese, die vorgesehen ist zum Fördern der Rekonstruktion in vivo eines Hohlorgans oder eines Teils eines Hohlorgans, **dadurch gekennzeichnet, dass** sie aufweist:
- eine biologisch abbaubare, rohrförmige, hohle Stütze, die wenigstens ein biokompatibles und biologisch abbaubares Polymer-Material aufweist, wobei die Stütze gebildet ist von einer äußeren, porösen Schicht und einer inneren, im Wesentlichen nicht porösen Schicht, und
- ein lebendes Material biologischen Ursprungs an der Außenfläche und/oder in wenigstens einem Teil der porösen Schicht der Stütze und/oder auf der Fläche der im Wesentlichen nicht porösen Schicht, die der porösen Schicht zugewandt ist, wobei das Material biologischen Ursprungs ausgewählt ist zum Erlauben der Rekonstruktion in vivo des Organs oder des Teils des Organs, wobei das Material biologischen Ursprungs nicht aus einem menschlichen Embryo(nen) entstammt.

2. Prothese gemäß Anspruch 1, **dadurch gekennzeichnet, dass** das Polymer-Material ausgewählt ist aus der Gruppe, die zusammengesetzt ist aus Chitosan, Chitin und deren Derivaten oder Copolymeren, insbesondere Chitin-Glucan, wobei diese Polymere gegebenenfalls mit wenigstens einem anderen biokompatiblen und biologisch abbaubaren Polymer verbunden sind.

3. Prothese gemäß Anspruch 2, **dadurch gekennzeichnet, dass** das wenigstens eine andere biokompatible und biologisch abbaubare Polymer ein Biopolymer ist, das ausgewählt ist aus der Gruppe, die zusammengesetzt ist aus Glykosaminoglykanen (GAG), insbesondere Hyaluron, Chondroitin-Sulfat oder Heparin, Kollagenen, Alginaten, Dextranen und Mischungen davon.

4. Prothese gemäß Anspruch 2, **dadurch gekennzeichnet, dass** das wenigstens eine andere biokompatible und biologisch abbaubare Polymer ein synthetisches biokompatibles und biologisch abbaubares Polymer ist, das aus der Gruppe ausgewählt ist, die zusammengesetzt ist aus biologisch abbaubaren Synthese-Polyestern wie z.B. Homopolymeren und Copolymeren basierend auf Milchsäure, Glycolsäure, Epsilon-Caprolacton und P-Dioxanon, oder noch ganz anderem, natürlichem Polyester, wie jenen der Familie Polyhydroxyalkanoate wie Homo- und Copolymere basierend auf Hydroxybutyrat, Hydroxyvalerate, Polyorthoestern, Polyurethanen.

5. Prothese gemäß einem der Ansprüche 1 bis 4, **dadurch gekennzeichnet, dass** der Porendurchmesser des porösen Teils größer als 10 µm ist, bevorzugt im Bereich zwischen 10 und 200 µm liegt.

6. Prothese gemäß einem der Ansprüche 1 bis 5, **dadurch gekennzeichnet, dass** das Hohlorgan eine Verdauungs-, Gallen-, Bauchspeicheldrüsen-, Harn-, Genital-, oder Blut-Röhre ist, insbesondere die Speiseröhre, der Darm, der Magen, der Choledochus, der Bauchspeicheldrüsengang, die Harnröhre, der Harnleiter, die Blase, der Eileiter, der Uterus und die Blutgefäße.

7. Prothese gemäß Anspruch 6, **dadurch gekennzeichnet, dass** das Hohlorgan die Speiseröhre ist.

8. Prothese gemäß einem der Ansprüche 1 bis 7, **dadurch gekennzeichnet, dass** das Material biologischen Ursprungs ein Material fötalen Ursprungs ist.

9. Prothese gemäß Anspruch 8, **dadurch gekennzeichnet, dass** das Material fötalen Ursprungs ein Organ, eine Organsegment oder eine Zellenemulsion fötalen Ursprungs ist.

10. Prothese gemäß Anspruch 9, **dadurch gekennzeichnet**, das Material fötalen Ursprungs in einer feuchten und viskosen Form ist, um seine Adhäsion an der Fläche und/oder in der Stütze zu verbessern.

11. Verfahren zur Herstellung einer Prothese gemäß einem der Ansprüche 1 bis 10, **dadurch gekennzeichnet, dass** es aufweist die Präparation einer biologisch abbaubaren, rohrförmigen Stütze, welche aufweist einer äußere, poröse Schicht, welche eine Zell-Proliferation erlaubt, wobei die äußere, poröse Schicht wenigstens ein biokompatibles und biologisch abbaubares Material aufweist, und eine innere, im Wesentlichen nicht poröse Schicht, und die Inkorporation eines biologischen Materials, das dazu bestimmt ist, um Prothese auszubilden, an der Außenfläche und/oder in wenigstens einem Teil der porösen Schicht der Stütze und/oder auf der Fläche der im Wesentlichen nicht porösen Schicht, die der porösen Schicht zugewandt ist, wobei das Material biologischen Ursprungs nicht aus einem menschlichen Embryo(nen) entstammt.

12. Verfahren gemäß Anspruch 11, **dadurch gekennzeichnet, dass** die Präparation der äußeren, porösen Schicht durch Lyophilisation erreicht wird.

13. Rohrförmige, biologisch abbaubare Stütze wie definiert in einem der Ansprüche 1 bis 7 für die Rekonstruktion wenigstens eines Teiles eines Hohlorgans.

14. Rohrförmige, biologisch abbaubare Stütze gemäß Anspruch 13, **dadurch gekennzeichnet, dass** das Polymer-Material ausgewählt ist aus der Gruppe, die zusammengesetzt ist aus Chitosan, Chitin und deren Derivaten oder Copolymeren, insbesondere Chitin-Glucan, wobei diese Polymere gegebenenfalls mit wenigstens einem anderen biokompatiblen und biologisch abbaubaren Polymer verbunden sind.

15. Rohrförmige, biologisch abbaubare Stütze gemäß Anspruch 13 oder 14, **dadurch gekennzeichnet, dass** der Porendurchmesser des porösen Teils größer als 10 µm ist, bevorzugt im Bereich zwischen 10 und 200 µm liegt.

16. Rohrförmige, biologisch abbaubare Stütze gemäß einem der Ansprüche 13 bis 15, **dadurch gekennzeichnet, dass** das Hohlorgan eine Verdauungs-, Gallen-, Bauchspeicheldrüsen-, Harn-, Genital-, oder Blut-Röhre ist, insbesondere die Speiseröhre, der Darm, der Magen, der Choledochus, der Bauchspeicheldrüsengang, die Harnröhre, der Harnleiter, die Blase, der Eileiter, der Uterus und die Blutgefäße.

17. Rohrförmige, biologisch abbaubare Stütze gemäß einem der Ansprüche 13 bis 16, **dadurch gekennzeichnet, dass** das Hohlorgan die Speiseröhre ist.

18. Verwendung einer rohrförmigen Stütze gemäß einem der Ansprüche 13 bis 17 für die Herstellung einer Prothese für die Regeneration eines Hohlorgans.

19. Verwendung gemäß Anspruch 18 für die Regeneration von Teilen der Speiseröhre, die eine Krankheit aufweisen.

20. Verwendung gemäß irgendeinem der Ansprüche 18 bis 19, **dadurch gekennzeichnet, dass** das Hohlorgan einen Teil aufweist, der durch irgendeine Krankheit oder einen Krebs beeinträchtigt ist.

## Claims

1. A prosthesis for promoting the in vivo reconstruction of a hollow organ or of a portion of a hollow organ, wherein said prosthesis comprises:
- a biodegradable hollow tubular support member comprising at least one biocompatible and biodegradable polymer material, said support member being constituted of a porous outer layer and an essentially non-porous inner layer; and
- a material of living biological origin at the outer surface, or within at least one portion of the porous layer of said support member, or over the surface of the essentially non-porous layer facing the porous layer, said material of biological origin allowing the in vivo reconstruction of said organ or of said organ portion, said material of biological origin not coming from human embryo(s).

2. The prosthesis according to claim 1, wherein said polymer material is selected from the group consisting of chitosan, chitin, and from derivatives or copolymers thereof optionally combined with at least one other biocompatible and biodegradable polymer.

3. The prosthesis according toclaim 2, wherein said at least one other biocompatible and biodegradable polymer is a biopolymer selected from the group consisting of glycosaminoglycans (GAGs), hyaluronan, chondroitin sulphate, heparin, collagens, alginates, dextrans and mixtures thereof.

4. The prosthesis according to claim 2, wherein said at least one other biocompatible and biodegradable polymer is a biocompatible and biodegradable synthetic polymer selected from the group consisting of synthetic biodegradable polyesters, homopolymers and copolymers based on lactic acid, glycolic acid, epsilon-caprolactone and p-dioxanone, or even a natural polyester usch as those of the familly of poly-hydroxyalkanoate, such as homo- and copolymers based on hydroxybutyrate, hydroxyvalerate, polyorthoester, polyurethane.

5. The prosthesis according to any one of claims 1 to 4, wherein the diameter of the pores of the porous portion is greater than 10 µm, preferably comprised between 10 and 200µm.

6. The prosthesis according to any one of claims 1 to 5, wherein said hollow organ is a digestive duct, biliary duct, urinary duct, genital duct, blood duct, in particular oesophagus, intestine, stomach, common bile duct, pancreatic duct, urethra, ureter, bladder, Fallopian tubes, uterus and blood vessels.

7. The prosthesis according to claim 6, wherein said hollow organ is the oesophagus.

8. The prosthesis according to any one of claims 1 to 7, wherein said material of biological origin is a material of fetal origin.

9. The prosthesis according to claim 8, wherein said material of fetal origin is an organ, an organ segment, or an emulsion of cells of fetal origin.

10. The prosthesis according to claim 9, wherein said material of fetal origin is in a wet and viscous form, so as to improve its adhesion to the surface of or within said support.

11. A process for manufacturing a prosthesis according to any one of claims 1 to 10, wherein said process comprises the preparation of a biodegradable tubular support member comprising a porous outer layer that enables cell proliferation, said porous outer layer comprising at least one biocompatible and biodegradable polymer material, and an essentially non-porous inner layer, and the incorporation of a biological material intended to form a prosthesis at the outer surface, or within at least one portion of the porous layer of said support member, or on the surface of the essentially non-porous layer facing the porous layer, said material of biological origin not coming from human embryo(s).

12. The process according to claim 11, wherein the preparation of the outer porous layer is carried out by lyophilization.

13. A biodegradable hollow tubular support as defined in any one of claims 1 to 7, for the reconstruction of at least one of a portion of a hollow organ.

14. The biodegradable hollow tubular support according to claim 13, wherein the polymer is selected from the group consisting of chitosan, chitin, and their derivatives or copolymers, in particular chitin-glucan, these polymers being optionally combined with at least one other biocompatible and biodegradable polymer.

15. The biodegradable hollow tubular support according to claim 13 or 14, wherein the pore diameter of the porous portion is greater than 10µm, and preferably comprised between 10 and 200µm.

16. The biodegradable hollow tubular support according to any one of claims 13 to 15, wherein said hollow organ is a digestive duct, biliary duct, urinary duct, genital duct, blood duct, in particular oesophagus, intestine, stomach, common bile duct, pancreatic duct, urethra, ureter, bladder, Fallopian tubes, uterus and blood vessels.

17. The biodegradable hollow tubular support according to any one of claims 13 to 16, wherein said hollow organ is oesophagus.

18. Use of a tubular support member according to any one of claim 13 to 17, for manufacturing prosthesis for the regeneration of a hollow organ.

19. Use according to claim 18, for the regeneration of portions of esophagus exhibiting a pathology.

20. Use according to any one of claims 18 to 19, wherein the hollow organ comprises a portion affected by any pathology or by a cancer.
